(19) Europäisches Patentamt
European Patent Office
Office européen des brevets

(11) Veröffentlichungsnummer: **0 181 515**
**B1**

(12) # EUROPÄISCHE PATENTSCHRIFT

(45) Veröffentlichungstag der Patentschrift: **05.09.90**

(51) Int. Cl.[5]: **A 61 K 9/32**

(21) Anmeldenummer: **85113044.3**

(22) Anmeldetag: **15.10.85**

(54) **Verfahren zur Herstellung einer wässrigen Überzugsmitteldispersion und ihre Verwendung zum Überziehen von Arzneimitteln.**

(30) Priorität: **19.10.84 DE 3438291**

(43) Veröffentlichungstag der Anmeldung:
**21.05.86 Patentblatt 86/21**

(45) Bekanntmachung des Hinweises auf die Patenterteilung:
**05.09.90 Patentblatt 90/36**

(84) Benannte Vertragsstaaten:
**AT BE CH DE FR GB IT LI NL SE**

(56) Entgegenhaltungen:
**EP-A-0 058 765**
**EP-A-0 088 951**

(73) Patentinhaber: **Röhm GmbH**
**Kirschenallee Postfach 4242**
**D-6100 Darmstadt 1 (DE)**

(72) Erfinder: **Lehmann, Klaus, Dr.**
**Schillerstrasse 85**
**D-6101 Rossdorf 1 (DE)**
Erfinder: **Dreher, Dieter**
**Hermannstädter Weg 34**
**D-6100 Darmstadt (DE)**
Erfinder: **Weisbrod, Wolfgang**
**Rodolf-Marburg-Strasse 11**
**D-6120 Michelstadt (DE)**

**EP 0 181 515 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft die Herstellung von filmbildenden wäßrigen Dispersionen von Überzugs- und Bindemitteln für feste Arzneimittel durch Dispergieren eines pulverförmigen Überzugsmittels in einer wäßrigen Phase unter Rühren. Die Erfindung betrifft weiterhin die dadurch hergestellten wäßrigen Dispersionen sowie ihre Verwendung zum Überziehen von Arzneiformen, und zum Granulieren von pharmazeutischen Wirkstoffpulvern und zur Herstellung von pharmazeutischen Filmen.

Aus der DE—OS 32 08 791 (entspr. EP—A—88 914) sind wäßrige Überzugsmitteldispersionen für Arzneiformen bekannt, die hergestellt worden sind, indem man ein pulverförmiges Emulsionspolymerisat, das saure oder basische Gruppen enthält, in einer Wasserphase, in der die sauren oder basischen Gruppen zu einem kleinen Teil durch eine Base oder Säure in die Salzform übergeführt werden, reemulgiert. Dabei werden die ursprünglichen Latexteilchen vereinzelt und durch oberflächliche Salzbildung hydratisiert und stabil dispergiert. Wegen des Gehalts an sauren oder basischen Gruppen in dem Überzugsmittel hängt die Löslichkeit oder Durchlässigkeit der daraus hergestellten Arzneimiztelüberzüge in den Verdauungssäften von deren pH-Wert ab. Eine solche Abhängigkeit vom pH-Wert kann—je nach der beabsichtigten Wirkungsweise des Arzneimittels—erwünscht oder unerwünscht sein.

Arzneimittelüberzüge mit einer vom pH-Wert des umgebenden wäßrigen Mediums unabhängigen Durchlässigkeit für gelöste Wirkstoffe werden aus Dragierlacken gemäß der DE—PS 1 617 751 hergestellt. Sie enthalten ein Mischpolymerisat mit quartären Ammoniumsalzgruppe und sind in organischen Lösungsmitteln, wie Alkoholen, Ketonen oder Estern, gelöst. Wegen des Gehalts an quartären Ammoniumgruppen ist es nicht möglich, derartige Polymerisate durch Emulsionspolymerisation herzustellen und daraus ein redispergierbares Pulver zu erzeugen.

Zum Überziehen von Arzneimitteln ist eine beim Trocknen filmbildende wäßrige Überzugsmitteldispersion zur Verfügung zu stellen, die wasserunlösliche Überzüge mit einer vom pH-Wert des umgebenden Mediums unabhängige Diffusionsdurchlässigkeit aufweist.

Es wurde gefunden, daß sich eine solche Dispersion durch Dispergieren eines pulverförmigen Überzugsmittels in einer wäßriger Phase unter Rühren bei erhöhter Temperatur herstellen läßt, wenn man als pulverförmige Überzugsmittel ein in Wasser quellbares, aber nicht lösliches Mischpolymerisat aus einer monoäthylenisch ungesättigten, radikalisch polymerisierbaren quartären Ammoniumverbindung und wenigstens einem nichtionischen, wasserunlösliche Homopolymerisate bildenden, monoäthylenisch ungesättigten, radikalisch polymerisierbaren Comonomeren in der wäßrigen Phase dispergiert.

Typische Dispersionen dieser Art enthalten, bezogen auf ihr Gesamtgewicht, 60 bis 85, vorzugsweise 70 bis 85 Gew.-% einer wäßrigen Phase und 40 bis 15, vorzugsweise 30 bis 15 Gew.-% eines darin dispergierten Mischpolymerisats aus 5 bis 20%, bezogen auf das Gewicht des Mischpolymerisats, der erwähnten quartären Ammoniumverbindung und 95 bis 70% des erwähnten nichtionischen Comonomeren und gegebenenfalls bis zu 20% anderen, ungesättigten, mit den vorgenannten Monomeren mischpolymerisierbaren Comonomeren.

Die neuen Überzugsmitteldispersionen enthalten keine flüchtigen brennbaren Bestandteile und lassen sich daher ohne Feuer- oder Explosionsgefahr und ohne Belastung der Abluft mit Fremdstoffen außer Wasserdampf zu klaren Filmen trocknen, die den aus organischer Lösung der gleichen Mischpolymerisate erzeugten Überzügen gleichwertig sind. Sie sind bei jedem im physiologischen Bereich vorkommenden pH-Wert in Wasser unlöslich, aber in solchem Maße quellbar, daß Wasser und darin gelöste pharmazeutische Wirkstoffe hindurchzudiffundieren vermögen. Ebenso wie die Quellbarkeit ist die Diffusionsdurchlässigkeit vom pH-Wert unabhängig, es sei denn, daß sich die Löslichkeit oder Diffusionsfähigkeit des Wirkstoffes selbst mit dem pH-Wert ändern. Die pH-unabhängige Durchlässigkeit beruht einerseits auf den quartären Ammoniumsalzgruppen, die als Salze einer starken Base im gesamten physiologischen pH-Bereich vollständig dissoziiert sind, andererseits auf der Abwesenheit anderer ionischer Gruppen, deren pH-abhängige Dissoziation eine pH-abhängige Diffusionsdurchlässigkeit nach sich ziehen würde.

Die neuen Dispersionen eignen sich daher zur Herstellung von pharmazeutischen Retardpräparaten, die den eingeschlossen Wirkstoff beim Durchgang durch den Verdauungskanal in jedem Bereich unabhängig vom herrschenden pH-Wert mit gleichgroßer Diffusionsgeschwindigkeit freisetzen und nach vollständiger Auslaugung unzersetzt ausgeschieden werden. Solche Polymerschichten können eine Hülle auf einer Tablette, einem Dragee, einer Kapsel oder einem Wirkstoffteilchen oder die Matrix eines Granulats oder einer daraus durch Verpressen hergestellten Gerüsttablette bilden oder auch einen Wirkstoff in dem Polymerfilm eingebettet enthalten. Die Hülle kann eine Dicke von 10 bis 50 µm haben und gegebenenfalls eine Schicht in einem mehrschichtigen Überzug bilden. Die Herstellung von Filmschichten, überzogenen Arzneiformen oder von Granulaten kann in der gleichen Weise wie mit anderen, bekannten wäßrigen Überzugsmitteldispersionen erfolgen. Sie werden häufig vor der Anwendung mit Füllstoffen oder Pigmenten, wie Talkum oder Titandioxid, mit Weichmachern und gegebenenfalls Geruchs- und Geschmackszusätzen modifiziert.

Ein besonderer Vorteil liegt in der Möglichkeit für den Verarbeiter der Überzugsmitteldispersionen, durch Mischen von zwei Dispersionen mit unterscheidlichem Gehalt an quartären Ammoniumgruppen jede gewünschte Permeabilität und

damit Freisetzungsgeschwindigkeit zwischen den Grenzwerten einstellen zu können, die sich bei Verwendung der beiden Komponenten allein ergeben. Da schon geringe Unterschiede im Aufbau der Mischpolymerisate deutliche Unterschiede der Freisetzungsgeschwindigkeit ergeben, erschließt sich durch Abmischung ein breiter Bereich von Retardwirkungen. So ließ z.B. ein Überzug aus einem Mischpolymerisat aus Äthylacrylat, Methylmethacrylat und Methacryloxyäthyl-trimethylammonium-chlorid im Gewichtsverhältnis 60/35/10. der zu Meßzwecken auf die Papiermembran einer Diffusionszelle aufgebracht war, innerhalb 5 Stunden 10,5 Teile Phenylpropanolamin-hydrochlorid durchdiffundieren, während ein sonst gleicher Überzug eines Mischpolymerisats mit dem Gewichtsverhältnis 64/35/5 der gleichen Monomerbestandteile unter gleichen Bedingungen eine Diffusion von nur 1,2 Teilen des genannten Wirkstoffes ergab. Mischungen der beiden Dispersionen ergaben bei den gleichen Versuchsbedingungen Diffusionswerte, die zwischen den beiden Extremwerten liegen. Die beigefügte Figur zeigt den Diffusionsverlauf innerhalb von 5 Stunden. Sie läßt außer der guten Einstellbarkeit der Diffusionsgeschwindigkeit auch die weitgehend lineare Diffusionsgeschwindigkeit bei jeder gewählten Zusammensetzung der Diffusionsschicht erkennen. Überzüge auf Arzneiformen verhalten sich in der gleichen Weise.

Der Aufbau des Mischpolymerisats

Für das Diffusionsverhalten ist die Art und vor allem der Mengenanteil des quartären Ammonium-Monomers von entscheidender Bedeutung. Als polymerisierbare quartäre Ammonium-Verbindungen kommen z.B. N-Vinyl-pyridiniumsalze in Betracht. Bevorzugt sind quartäre Aminoalkylester oder Aminoalkylamide der Acryl- oder Methacrylsäure. Sie entsprechenden der allgemeinen Formel

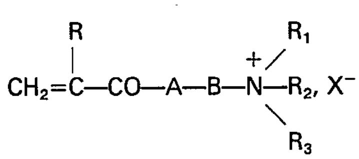

worin

R ein Wasserstoffatom oder eine Methylgruppe

A ein Sauerstoffatom oder ein NH-Gruppe

B ein geradkettiger oder verzweigter, gegebenenfalls alicyclischer Alkylrest, insbesondere mit 2 bis 8 C-Atomen.

$R_1$, $R_2$ und $R_3$ gleich oder verschiedene Alkyl- oder Aralkylreste, insbesondere niedere Alkylreste mit 1 bis 4 C-Atomen oder Benzylreste, bedeuten oder gegebenenfalls $R_1$ und $R_2$ zusammen mit dem quartären N-Atom einen Piperdinium- oder Morpholiniumrest bilden,

$X^-$ ist ein Äquivalent eines—vorzugsweise anorganischen—Säureanions, insbesondere Chlorid, Sulfat oder Methosulfat.

Als Beispiele dieser Verbindungsklasse seien Acryl- und Methacryloxy-trimethylammonium-chlorid und -methosulfat, Benzyldimethylammoniumäthyl-methacrylat-chlorid, Diäthylmethylammoniumäthyl-acrylat- und -methacrylat-methosulfat, N-Trimethylammoniumpropyl-methacrylamid-chlorid und N-Trimethylammonium-2,2-dimethyl-propyl-1-methacrylat-chlorid genannt.

Der Anteil dieser Monomeren am Aufbau des Mischpolymerisats liegt in der Regel bei 5 bis 20 Gew.-%. Geringere Anteile lassen keine ausreichende Diffusionsdurchlässigkeit mehr erreichen. Höhere Anteile machen das Mischpolymerisat wasserlöslich. Der bevorzugte Gehalt liegt bei 5 bis 10 Gew.-%. Der Anteil dieser Monomeren wird mit Vorteil so festgelegt, daß das feinteilige Polymerisat in reinem Wasser durch Quellung 10 bis 100, vorzugsweise 20 bis 80 Gew.-% Wasser aufnimmt. Zum Abmischen lassen sich beispielsweise zwei Mischpolymerisate verwenden, von denen das eine 10 bis 40 und das andere 50 bis 80 Gew.-% Wasser aufnimmt.

Als wasserunlösliche Homopolymerisate bildende, nichtionische Comonomere kommen vor allem die Alkylester der Acryl- und Methacrylsäure, Styrol und dessen Homologe, Vinylester und Vinylchlorid in Betracht. Unter nichtionischen Monomeren werden nicht nur solche verstanden, die keine ionischen Gruppen, wie Alkalicarboxylat-, Alkali-sulfonat- oder tert.- Ammonium-Gruppen, im Molekül enthalten, sondern auch solche, die nicht mit Basen bzw. Säuren Gruppen zu bilden vermögen, wie ungesättigte Säuren und Amine. Die Alkylester der Acryl- und Methacrylsäure mit 1 bis 8 C-Atomen im Alkylrest sind bevorzugt.

Die Härte und Dehnbarkeit des Überzugsfilms und die Mindesttemperatur, bei der eine Filmbildung aus der Disperions möglich ist, werden durch diese Monomerkomponente wesentlich beeinflußt. Durch Ester der Acrylsäure wird die Filmhärte vermindert, seine Elastizität und Dehnbarkeit erhöht und die Filmbildungstemperatur gesenkt. Da Acrylester oder die höheren Alkylester der Methacrylsäure meistens zu weiche und klebrige Filme ergeben, wenn sie als einzige nichtionische Comonomere verwendet werden, verwendet man sie vorzugsweise in Kombination mit Monomeren, die selbst härtere Homopolymerisate bilden, z.B. Styrol und die niederen Alkylester der Methacrylsäure. Diese Ester ergeben allein meistens zu harte Filme oder eine zu hohe Filmbildungstemperatur. Höhere Alkylreste (mit mehr als 4 C-Atomen) oder Arylreste wirken hydrophobierend und setzen dadurch die Quellfähigkeit und Diffusionsdurchlässigkeit herab. Comonomere mit derartigen Gruppen werden daher höchstens dann mitverwendet, wenn ein solcher Effekt beabsichtigt ist.

Die vorteilhaften Eigenschaften der erfindungsgemäßen Dispersionen oder der daraus herstellbaren überzüge können allein mit fen beiden oben beschriebenen Monomerkomponenten—nämlich den quartären Ammonium- und den nichtionischen Monomeren—herbeigeführt werden. Es können jedoch in vielen Fällen auch

andere Monomere am Aufbau des Mischpolymerisats beteiligt sein, ohne die Ziele der Erfindung zu gefährden. Allerdings sollen ionische Monomere—im oben beschriebenen Sinne—möglichst einen Anteil von 1 bis 2 Gew.-% des Mischpolymerisats nicht überschreiten. Geringe Mengen an ionischen Monomeren, wie Acryl- oder Methacrylsäure oder die den quaternierten Monomeren zugrundeligenden Aminomonomeren, sind gelegentlich als Verunreinigungen der Ausgangsmonomeren schwierig auszuschließen. Wasserlösliche nichtionische Comonomere, wie Acryl- oder Methacrylamid, Vinylpyrrolidon oder Hydroxyalkylester der Acryl- oder Methacrylsäure, können das Quell- und Diffusionsverhalten spürbar beeinflussen, lassen jedoch die pH-Unabhängigkeit dieses Verhaltens unberührt. Der Anteil solcher Monomerer ist auf höchstens 20% des Mischpolymerisats beschränkt, liegt aber vorzugsweise unter 10 oder sogar unter 5%. Die Hydroxyalkylester erweisen sich häufig als nachteilig und werden vorzugsweise gänzlich vermieden. Ebenso sollen mehrfach äthylenisch ungesättigte Monomere, die zur Vernetzung führen, auch in kleinen Mengen nicht mitverwendet werden.

Die Herstellung des Mischpolymerisats ist nich Gegenstand der Erfindung und kann nach beliebigen, dafür geeigneten Methoden erfolgen. Am einfachsten ist die Polymerisation in Substanz in Gegenwart eines in dem Monomerengemisch gelösten radikalbildenden Initiators. Man kann auch eine Lösungs- oder Fällungspolymerisation in einem organischen Lösungsmittel durchführen und das entstandene Polymerisat von dem Lösungsmittel abtrennen. Wichtig ist in allen Fällen, daß das Mischpolymerisat in Form eines feinen Pulvers erhalten wird, was bei Substanzpolymerisaten durch Mahlen, bei Lösungs- oder Fällungspolymerisaten durch Sprühtrocknung erreichbar ist. Die Pulverteilchen sollen eine solche Größe haben, daß sie ein Sieb mit einer lichten Maschenbreite von 200 µm passieren. Korngrößen unter 20 bis 50 µm sind verwendbar, aber weger der Neigung zum Stauben weniger zweckmäßig.

Herstellung der dispersion

Die bloße Dispergierung des pulverförmigen Mischpolymerisats in Wasser führt in der Regel noch nicht zu einer beständigen, filmbildenden Dispersion. Überraschenderweise bilden sich bei längerem Rühren bei erhöhter Temperatur selbstemulgierbare Teilchen, deren Größe weit unter der der eingesetzten Pulverkörnchen liegt. Ihre mittlere Teilchengröße (Gewichtsmittelwert) kann im Bereich von 1 bis 50 µm liegen und beträgt vorzugsweise 10 bis 20 µm. Um die stabile Dispergierung des Mischpolymerisats zu erreichen, genügt es, die Pulverteilchen durch Rühren in der Schwebe zu halten und die Bildung eines Bodensatzes zu verhindern. Dazu reichen niedrige bis mäßige Rührgeschwindigkeit in üblichen Rührkesseln aus. Das Rühren wird solange fortgesetzt, bis sich beim Stehen der Dispersion kein Bodensatz mehr bildet. Um eine vollständige Emulgierung zu erreichen, ist in der Regel eine Rührzeit von mindestens 15 Minuten, vorzugszweise mehr als 20 Minuten, beispielsweise 30 bis 60 Minuten erforderlich. Der Zusatz von Emulgatoren ist entbehrlich. Längere Rührzeiten oder höhere Rührgeschwindigkeiten stoßen höchstens durch eine allmählich zunehmende Schaumbildung an Grenzen.

Die Dispergierung wird gefördert, wenn schon zu Beginn ein Weichmachungsmittel für das Mischpolymerisat zugesetzt wird. Weichmachermengen von 10 bis 20%, bezogen auf das Gewicht des Mischpolymerisats, sind beim Dispergiervorgang selbst wirksam und verbessern die Filmbildungsfähigkeit bei der Herstellung von Überzügen. Als Weichmachungsmittel eignen sich nicht-flüchtige, mit dem Mischpolymerisat verträgliche, physiologisch unbedenkliche Zusätze mit einem meistens unter 1000 liegenden Molekulargewicht. Überwiegend handelt es sich um flüssige oder amorph erstarrende, leicht aufschmelzbare Stoffe, wie z.B. Polyäthylenglykole, Citronensäureester von niederen Alkoholen, Fettsäureester von Zuckeralkoholen oder deren Oxäthylierungsprodukte, von denen (gegebenenfalls oxäthyliertes) Sorbitanmonooleat der bekannteste Vertreter ist.

Dispersionen, die ohne Weichmacherzusatz erst bei 40 oder 50°C flimbildend sind, können mit dem Weichmacherzusatz schon bei 0 bis 20°C zu klaren und homogenen Überzügen verarbeitet werden.

Die beim Dispergieren erforderliche Temperatur hängt von der Polymerisathärte ab und muß mit steigender Härte höher gewählt werden. Temperturen von 50 bis 100°C, vorzugsweise 60 bis 80°C sind im allgemeinen geeignet. Das Mengenverhältnis der Pulvermenge zur Menge der wäßrigen Phase wird so abgestimmt, daß eine gut filmbildende Dispersion entsteht. Die Filmbildungsfähigkeit unter den Bedingungen technischer Beschichtungsverfahren ist vom Feststoffgehalt der Dispersion abhängig. Wenn der Feststoffgehalt zu niedrig ist, wird kein gleichmäßiger Film erzeugt oder es wird eine unverhältnismäßig lange Trockenzeit benötigt. In der Regel besteht gute Filmbildungsfähigkeit bei einem Anteil des Mischpolymerisats von 15 bis 40, vorzugsweise 15 bis 30 Gew.-% und dementsprechend 85 bis 60, vorzugsweise 85 bis 70 Gew.-% der wäßrigen Phase.

Dispersionen mit einem noch höheren Feststoffgehalt sind wegen der zunehmenden Viskosität schwer herstellbar und aus dem gleichen Grund schlecht verarbeitbar.

Obwohl es möglich ist, die erzeugten stabilen Dispersionen in großen Vorratsmengen herzustellen, zu lagern oder zu vertreiben, ist es für den Verarbeiter vorteilhafter, jeweils für den kurzfristig absehbaren Bedarf das Pulverförmige Überzugsmittel selbst zu dispergieren. Es ist dadurch möglich, je nach der im Einzelfall erwünschten Retardwirkung mehrere Mischpolymerisatpulver von unterschiedlicher Quellfähigkeit im erforderlichen Mischungsverhältnis einzusetzen und die

Dispersion im gleichen Arbeitsgang mit Weichmachungsmitteln und weiteren Zusätzen nach Bedarf zu konfektionieren.

Die Herstellung von Überzügen auf Arzneiformen mit den erfindungsgemäßen Überzugsmitteldispersionen erfolgt in an sich bekannter Weise im Kesseldragierverfahren oder im Wirbelschichtverfahren. Wirkstoffpulver oder -Kristalle können in herkömmlicher Weise granuliert werden. Die bevorzugte Anwendung besteht in der Herstellung von Endüberzügen von 10 bis 50 µm Dicke auf Pellets oder Granulaten, die z.B. in Hartgelatinekapseln abgefüllt werden könne. Auch kann man die Dispersionen zur Herstellung von dermalen oder transdermalen Arzneiformen verwenden, indem man Wirkstoffe in Filmschichten einbettet und die Filme auf Papiervliese oder inerte Kunststoffolien aufträgt oder freitragende Filmschichten herstellt.

Die Dispersion werden bei Temperaturen unter 80°, im allgemeinen nicht über 60°C verarbeitet. Bevorzugt sind Temperaturen unter 40°C, beipielsweise bei 20 bis 30°C. Bei der Konfektionierung ist anzustreben, Überzüge oder Filme herzustellen, die bis zu Temperaturen von 30 bis 35°C nicht klebrig werden.

Beispiel 1

350 g Wasser wurden in einem Kolben unter Rühren auf 65°C erwärmt und innerhalb von etwa 10 Minuten 150 g eines feingemahlenen Substanzpolymerisats aus 60 Gew.-Teilen Methylmethacrylat, 30 Gew.-Teilen Äthylacrylat und 10 Gew.-Teilen Trimethylammoniumäthylmethacrylatchlorid eingetragen. Das Polymerpulver war vorher über ein Sieb mit 0,2 mm lichter Maschenweite gesiebt worden. Die Rührgeschwindigkeit wurde so einreguliert, daß sich die Partikel am Boden oder den Wandungen des Kolbens nicht festsetzen konnten. Die Temperatur lag im Bereich von 65—75°C. Nach 2 Stunden war eine milchige, leicht viskose Dispersion entstanden, die durch ein Sieb mit 0,1 mm Maschenweite passiert wurde. Der Rückstand betrug weniger als 3 g, so daß mehr als 98% des Polymeren dispergiert waren. Der Trockengehalt der Dispersion betrug 30,1%.

100 g der Dispersion wurden mit 3 g Zitronensäuretriäthylester als Weichmacher versetzt. Oberhalb einer Mindestfilmbildetemperatur von 17°C bilden sich aus der auf einer PVC-Platte ausgestrichenen Dispersion klare, leich abziehbare Filme.

Beispiel 2

300 g einer nach Beispiel 1 hergestellten wäßrigen Dispersion mit 30% Trockensubstanzgehalt wurden mit 800 g einer Pigmentsuspension aus 125 g Talkum, 55 g Titandioxid, 25 g Gelblack E 104, 25 g Polyäthylenglykol 6000 und 570 g Wasser vermischt und mit weiteren 500 g Wasser verdünnt, so daß sich ein Gesamtfeststoffgehalt der Lackpigmentsuspension von 20% ergab. In einem VA-Stahl-Dragierkessel von 50 cm Durchmesser wurden nun 10 kg Tabletten (Durchmesser 7 mm, Höhe 3,5 mm. Gewicht 140 mg/Stück) durch Einblasen von Warmluft auf etwa 35°C vorgewärmt und mit einer Luftdrucksprühpistole mit einer Düse von 1,5 mm Durchmesser bei einem Luftdruck von etwa 1 bar ein feiner Sprühstrahl auf die im Dragierkessel rotierenden Kerne gerichtet. Die Sprühgeschwindigkeit wurde bei kontinuierlichem Einblasen von Trockenluft auf 25—30 g/Minute gehalten, wobei die Temperatur der eingeblasenen Warmluft zwischen 40 und 60°C lag. Die Tablettenkerne hatten bei diesen Arbeitsbedingungen eine Oberflächentemperatur von etwa 25°C. Nachdem der gesamte Sprühauftrag innerhalb von 90 Minuten abgeschlossen war, wurde noch etwa 5 Minuten bei langsamer Rotation des Kessels Warmluft eingeblasen. Danach wurden die überzogenen Tabletten auf Filterpapier ausgebreitet und über Nacht an der Luft nachgetrocknet. Es entstand ein gleichmäßig gefärbter, glänzender Überzug. Die überzogenen Tabletten sind staubfrei und lassen sich leicht schlucken. In Wasser, künstlichen Magensaft und wäßrigen Pufferlösungen tritt innerhalb von 10 Minuten ein schnelles Aufquellen und Ablösen des Filmüberzugs ein, so daß die Tabletteninhaltsstoffe schnell freigegeben werden.

Beispiel 3

350 g Wasser wurden in einem 500 ml-Rundkolben zunächst mit 45 g Tween 80® versetzt und auf 80°C erwärmt, wobei eine klare Lösung entstand. Dann wurden zunächst 50 g eines Copolymerisats aus 65 Teilen Methylmethacrylat, 30 Teilen Äthylacrylat und 5 Teilen Trimethylammoniumäthylmethacrylatchlorid in Form eines körnigen Granulats mit einer Partikelgröße von etwa 1—2 mm eingetragen. Die Rührgeschwindigkeit wurde so einreguliert, daß sich keine Partikel an den Wandungen festsetzen konnten. Nach einer Stunde Rührzeit wurden nochmals 50 g des Polymerisates hinzugegeben und dieser Vorgang nach einer weiteren Stunde Rührzeit bei 80° nochmals wiederholt. Die Mischung wurde nun noch weitere 5 Stunden bei 80°C gerührt und über Nacht langsam abkühlen gelassen. Die milchige Dispersion wurde durch ein 0,1 mm Sieb passiert. Der Rückstand betrug weniger als 5 g. Der Trockengehalt der Dispersion betrugt 29%.

100 g dieser Dispersion wurden mit 3 g Triäthylzitronensäureester vermischt und ergaben beim Trocknen einer daraus gebildeten Schicht einen klaren, aber noch spröden Film. Setzt man weitere 3 g dieses Esters hinzu, so entsteht ein flexibler Film, wobei die Mindestfilmbildungstemperatur 2°C betrug.

Die Bestimmung der Teilchengrößenverteilung in der Ultrazentrifuge ergab eine mittlere Teilchengröße $r_w$ von 150—200 nm, wobei einige Partikel im Korngrößenbereich von 1 µm ebenfalls noch sichtbar waren.

Beispiel 4

350 g Wasser wurden auf 60°C erwärmt und hierin unter Rühren 5 g Citronensäuretriäthylester gelöst. Nun wurden 50 g eines sprühgetrockneten

Polymerisates aus 60 Gew.-Teilen Methylmethacrylat, 30 Gew.-Teilen Äthylacrylat und 10 Teile Trimethylammoniumäthylmethacrylatchlorid eingerührt. Innerhalb von 90 min entstand eine milchige Dispersion, die unter Rühren auf Raumtemperatur abgekühlt wurde.

Beispiel 5

400 g eines Theophyllin-Pulvers, Partikelgröße unter <0,2 mm, wurden mit 160 g sekundärem Calciumphosphat in einer Knetmaschine mit 400 g einer nach Beispiel 3 hergestellten und auf 15% Trockengehalt verdünnten Dispersion homogen befeuchtet und auf einer Granuliermaschine mit einem Sieb von 1,5 mm lichter Maschenweite granuliert. Anschließend wurde in einem Trokkenschrank bei 100°C bis auf eine Restfeuchte unter 2% getrocknet, anschließend noch einmal durch ein 1,5 mm Sieb passiert und mit 6,6 g Talkum und 3,6 g Mg-stearat als Gleitmittel in einem Doppelkonusmischer vermischt. Dieses Granulat wurde zu Tabletten von 12 mm Durchmesser und einem Gesamtgewicht von je 500 mg verpreßt, so daß der Wirkstoffgehalt 400 mg pro Tablette betrug. Bei einem Wirkstoffabgabetest in einem Paddlegerät nach USP XX (Apparatur Nr. 2) zeigten diese Tabletten eine über 6 Stunden verzögerte Wirkstoffabgabe.

Beispiel 6

1,5 kg Mikrodragées einer Korngröße von 0,5—1,2 mm Durchmesser mit einem Gehalt von 8% Chlorphenaminmaleat als Wirkstoff wurde in einem Wirbelschichtgerät (Uniglatt der Fa. Glatt, Binzen, BRD) in einem Warmluftstrom von 50°C aufgewirbelt und mit Hilfe einer Luftdruck-Sprühpistole mit einem Düsendurchmesser von 1,0 mm bei einem Sprühdruck von 1,8 bar mit einer Mischung aus 500 g einer nach Beispiel 3 hergestellten Dispersion mit 30% Trockengehalt, 30 g Triäthylzitronensäureester, 75 g Talkum und 700 g Wasser als Verdünnungsmittel innerhalb von 80 Minuten aufgesprüht. Nach dem Auftrag von 80% der obigen Rezeptur, der einem Lackanteil von 8%, bezogen auf das Gewicht der Ausgangspellets entspricht, wurden Proben entnommen und ebenso wie nach dem Gesamtlackauftrag von 10% in einem Paddle-Gerat nach USP XX, App. 2 untersucht. Es ergab sich hierbei eine verzögerte kontinuierliche Wirkstoffabgabe mit folgenden Werten:

8% Lackauftrag

| | |
|---|---|
| 1 Stunde — 7,5% | pH 1,5 |
| 2 Stunden—28,5% | pH 2,1 |
| 3 Stunden—55 % | pH 5.5 |
| 4 Stunden—73 % | pH 6.5 |
| 5 Stunden—84 % | pH 6.7 |
| 6 Stunden—92 % | pH 6.8 |

10% Lackauftrag

| | |
|---|---|
| 1 Stunde — 1.5% | pH 1,5 |
| 2 Stunden— 8,0% | pH 2,1 |
| 3 Stunden—19,5% | pH 5,5 |
| 4 Stunden—39,5% | pH 6,5 |
| 5 Stunden—59 % | pH 6,7 |
| 6 Stunden—71 % | pH 6,8 |

Als Lösemedien wurden Mischungen aus künstlichem Magensaft und künstlichem Darmsaft nach BP verwendet, wobei sich die angegebenen, von Stunde zu Stunde steigenden, pH-Werte ergaben.

Beispiel 7

Von den nach Beispiel 1 und 3 hergestellten Dispersionen mit jeweils 30% Trockengehalt wurden Mischungen im Verhältnis 8:2, 6:4, 5:5, 4:6 und 2:8 hergestellt. Außerdem wurden die unvermischten Dispersionen untersucht. Ein Papiervlies von etwa 15 µm Dicke wurde zunächst mit Wasser befeuchtet, überschüssige Flüssigkeit mit einem Gummiroller abgedrückt. Anschließend wurde auf dieses so vorbehandelte Papier so viel Dispersion aufgetragen, daß sich eine Filmschicht von 20 µm Dicke ausbildete. Nach dem vollständigen Trocknen wurde das beschichtete Papier in eine 12 $cm^2$ große Diffusionszelle des Sartorius-Resorptionsmodells Typ SM 16750 eingespannt, von der Lackseite mit einer 36 %igen Lösung von Phenylpropanolamin · HCl in Wasser und von der anderen Seite mit isotonischem Phosphatpuffer (nach Hagers Handbuch II, Erg. Bd. I. S. 125) pH 6,0 gespült. Es wurde nun spektralphotometrisch bei 262 nm die hindurchdiffundierende Wirkstoffmenge mit der Zeit verfolgt und die Meßpunkte nach jeweils 1, 2, 3, 4 und 5 Stunden in Abhängigkeit vom Mischungsverhältnis der beiden Dispersionen in das Diagramm Abb. 1 eingetragen. Es zeigt sich, daß die nach Beispiel 3 hergestellte Dispersion Filme mit sehr geringer Durchlässigkeit ergibt, während die Filme aus der nach Beispiel 1 hergestellten Dispersion eine sehr hohe Permeabilität zeigen. Die Ergebnise für die verschiedenen Mischungen liegen zwischen diesen beiden Extremwerten, so daß man mit Hilfe eines solchen Diagramms das für einen bestimmten Wirkstoff günstige Mischungsverhältnis bestimmen kann, um die Freisetzungsgeschwindigkeit den therapeutischen Efordernissen anzupassen.

Beispiel 8

10 g einer 10 %igen Lösung von Chlorphenaminmaleat in Wasser wurden mit 100 g einer nach Beispiel 3 hergestellten Dispersion mit 27% Trokkengehalt vermischt und außerdem noch 6 g Triäthylzitronensäureester als Weichmacher hinzugegeben. Auf Papier wurde nun wie in Beispiel 7 beschrieben, so viel Dispersion ausgestrichen, daß ein Film von etwa 115 µm Dicke entstand. Dieser wurde analog zu Beispiel 7 in einem Sartorius-Resorptionsmodell auf die Wirkstofffreigabe untersucht, wobei jedoch nur auf der Filmseite mit Puffer gespült wurde. Der Wirkstoff wurde innerhalb von mehr als 72 Stunden verzögert aus dem Film freigesetzt.

**Patentansprüche**

1. Verfahren zur Herstellung einer beim Trocknen filmbildenden Dispersion eines Überzugsmittels für Arzneimittel durch Dispergieren eines pulverförmigen Überzugsmittels in einer wäßrigen Phase unter Rühren bei erhöhter Temperatur, dadurch gekennzeichnet, daß ein pulverförmiges, in Wasser quellbares, aber nicht lösliches Mischpolymerisat aus einer monoäthylenisch ungesättigten, radikalisch polymerisierbaren quartären Ammoniumverbindung und wenigstens einem nichtionischen, wasserunlösliche Homopolymerisate bildenden, monoäthylenisch ungesättigten, radikalisch polymerisierbaren Comonomeren in der wäßrigen Phase dispergiert wird.

2. Verfahren nach Anspruch 1, dadurch gekennzeichnet, daß ein Mischpolymerisat eingesetzt wird, das aus

a) 5 bis 20 Gew.-% der quartären Ammoniumverbindung und

b) 95 bis 70 Gew.-% des wasserunlösliche Homopolymerisate bildenden Comonomeren aufgebaut ist.

3. Verfahren nach den Ansprüchen 1 oder 2, dadurch gekennzeichnet, daß ein Mischpolymerisat eingesetzt wird, an dessen Aufbau als quartäre Ammoniumverbindung ein quartärer Aminoalkylester oder ein quartäres Aminoalkylamid der Acryl- oder Methacrylsäure beteiligt ist.

4. Verfahren nach den Ansprüche 1 bis 3, dadurch gekennzeichnet, daß das pulverförmige Mischpolymerisat in einer Teilchengröße nicht über 200 µm eingesetzt wird.

5. Verfahren nach den Ansprüchen 1 bis 4, dadurch gekennzeichnet, daß zur Herstellung von 100 Gew.-Teilen der wäßrigen Dispersion 15 bis 40 Gew.-Teile des pulverförmigen Mischpolymerisats und 85 bis 60 Gew.-Teile der wäßrigen Phase eingesetzt werden.

6. Verfahren nach den Ansprüchen 1 bis 5, dadurch gekennzeichnet, daß zur Herstellung der Dispersion wenigstens 15 min gerührt wird.

7. Verfahren nach den Ansprüchen 1 bis 6, dadurch gekennzeichnet, daß die Dispersion durch Rühren bei einer Temperatur zwischen 50 und 100°C, vorzugsweise bei 60 bis 80°C hergestellt wird.

8. Verfahren nach den Ansprüchen 1 bis 7, dadurch gekennzeichnet, daß während oder nach der Herstellung der Dispersion wasserunlösliche Füllstoffe oder Pigmente zugesetzt werden.

9. Filmbildende wäßrige Überzugsmitteldispersion für Arzneimittel, enthaltend 60 bis 85 Gew.-% einer wäßrigen Phase und 40 bis 15 Gew.-% eines darin dispergierten, in Wasser quellbaren, aber nicht löslichen Mischpolymerisats aus 5 bis 20% (bezogen auf das Gewicht des Mischpolymerisats) einer monoäthylenisch ungesättigten, radikalisch polymerisation quartären Ammoniumverbindung, und 95 bis 70%, wenigstens eines nichtionischen, wasserunlösliche Homopolymerisate bildenden, monoäthylenisch ungesättigten polymerisierbaren Comonomeren und gegebenenfalls bis zu 20% anderen ungesättigten, mit den vorgenannten mischpolymerisierbaren Comonomeren.

10. Verwendung der nach den Ansprüche 1 bis 8 hergestellten Dispersion zum Überziehen von Arzneiformen oder zum Granulieren von pharmazeutischen Wirkstoffpulvern.

11. Verwendung der nach den Ansprüchen 1 bis 8 hergestellten Dispersion zur Herstellung einer Filmschicht mit eingebettem Wirkstoff durch Eintrocknen einer Schicht der mit dem Wirkstoff versetzten Dispersion.

**Revendications**

1. Procédé de préparation d'une dispersion filmogène au séchage d'un agent d'enrobage pour médicaments, par dispersion d'un agent d'enrobage pulvérulent dans une phase aqueuse sous agitation à température élevée, caractérisé en ce qu'un copolymère pulvérulent, gonflable mais non soluble dans l'eau, formé d'un composé d'ammonium quaternaire insaturé monoéthyléniquement, susceptible de polymérisation radicalaire, et d'au moins un comonomère non ionique insaturé monoéthyléniquement, susceptible de polymérisation radicalaire et formant des homopolymères insolubles dans l'eau, est dispersé dans la phase aqueuse.

2. Procédé selon la revendication 1, caractérisé en ce qu'on utilise un copolymère qui est constitué par

a) 5 à 20% en poids du composé d'ammonium quaternaire et

b) 95 à 70% en poids du comonomère formant des homopolymères insolubles dans l'eau.

3. Procédé selon la revendication 1 ou 2, caractérisé en ce qu'on utilise un copolymère à la constitution duquel a participé, en tant que composé d'ammonium quaternaire, un aminoalkylester quaternaire ou un aminoalkylamide quaternaire de l'acide acrylique ou méthacrylique.

4. Procédé selon l'une quelconque des revendications 1 à 3, caractérisé en ce que le copolymère pulvérulent est utilisé avec une grosseur de particules qui ne dépasse pas 200 µm.

5. Procédé selon l'une quelconque des revendications 1 à 4, caractérisé en ce que pour la préparation de 100 parties en poids de la dispersion aqueuse, on utilise de 15 à 40 parties en poids du compolymère pulvérulent et de 85 à 60 parties en poids de la phase aqueuse.

6. Procédé selon l'une quelconque des revendications 1 à 5, caractérisé en ce que l'agigation est maintenue pendant 15 mn au moins pour la préparation de la dispersion.

7. Procédé selon l'une quelconque des revendications 1 à 6, caractérisé en ce que la dispersion est préparée par agitation à une température comprise entre 50 et 100°C, de préférence entre 60 et 80°C.

8. Procédé selon l'une quelconque des revendications 1 à 7, caractérisé en ce que des matières de charge ou des pigments insolubles dans l'eau sont ajoutés pendant ou après la préparation de la dispersion.

9. Dispersion aqueuse filmogène d'agent d'enrobage pour médicaments, contenant de 60 à 85% en poids d'une phase aqueuse et, en dispersion dans celle-ci, de 40 à 15% en poids d'un copolymère gonflable mais non soluble dans l'eau, formé de 5 à 20% (par rapport au poids du copolymère) d'un composé d'ammonium quaternaire insaturé monoéthyléniquement, susceptible de polymérisation radicalaire, de 95 à 70% d'au moins un comonomère non ionique insaturé monoéthyléniquement, polymérisable et formant des homopolymères insolubles dans l'eau, et éventuellement de 20% au maximum d'autres comonomères insaturés, copolymérisables avec le comonomère précité.

10. Utilisation de la dispersion préparée selon l'une quelconque des revendications 1 à 8 pour l'enrobage de formes médicamenteuses ou pour la granulation de poudres de substances actives pharmaceutiques.

11. Utilisation de la dispersion préparée selon l'une quelconque des revendications 1 à 8 pour la confection d'une couche pelliculaire dans laquelle est incluse une substance active, par séchage d'une couche de la dispersion additionnée de la substance active.

**Claims**

1. Process for preparing a coating agent dispersion for medicaments which forms a film on drying, by dispersing a powder coating agent in an aqueous phase with stirring at elevated temperature, characterised in that a powder, water-swellable, but insoluble copolymer from a monoethylenically unsaturated, quaternary ammonium compound, which can be polymerised by free radicals, and at least one non-ionic, monoethylenically unsaturated comonomer, which can be polymerised by free radicals and which forms water-insoluble homopolymers, is dispersed in the aqueous phase.

2. Process according to Claim 1, characterised in that a copolymer is used which is composed of

a) 5 to 20 wt.% of the quaternary ammonium compound and

b) 95 to 70 wt.% of the comonomer forming water-insoluble homopolymers.

3. Process according to Claims 1 or 2, characterised in that a copolymer is used, in the structure of which a quaternary aminoalkyl ester or a quaternary aminoalkylamide of acrylic acid or methacrylic acid is incorporated as quaternary ammonium compound.

4. Process according to Claims 1 to 3, characterised in that the powder copolymer is used at a particle size not greater than 200 µm.

5. Process according to Claims 1 to 4, characterised in that 15 to 40 parts by weight of the powder copolymer and 85 to 60 parts by weight of the aqueous phase are used to prepare 100 parts by weight of the aqueous dispersion.

6. Process according to Claims 1 to 5, characterised in that stirring is carried out for at least 15 minutes to prepare the dispersion.

7. Process according to Claims 1 to 6, characterised in that the dispersion is prepared by stirring at a temperature between 50 and 100°C, preferably at 60 to 80°C.

8. Process according to Claims 1 to 7, characterised in that water-insoluble fillers or pigments are added during or after the preparation of the dispersion.

9. Film-forming, aqueous coating agent dispersion for medicaments containing 60 to 85 wt.% of an aqueous phase and 40 to 15 wt.% of a water-swellable, but insoluble copolymer from 5 to 20% (relative to the weight of the copolymer) of a monoethylenically unsaturated, quaternary ammonium compound, which can be polymerised by free radicals, dispersed therein, and 95 to 70% of at least one non-ionic monoethylenically unsaturated comonomer, which can be polymerised and which forms water-insoluble homopolymers, and optionally up to 20% of other unsaturated comonomers which can be copolymerised with those mentioned above.

10. Use of the dispersion prepared according to Claims 1 to 8 for coating medicament shapes or for granulating pharmaceutical active ingredient powders.

11. Use of the dispersion prepared according to Claims 1 to 8 for the preparation of a film layer having embedded active ingredient, by drying a layer of the dispersion treated with the active ingredient.

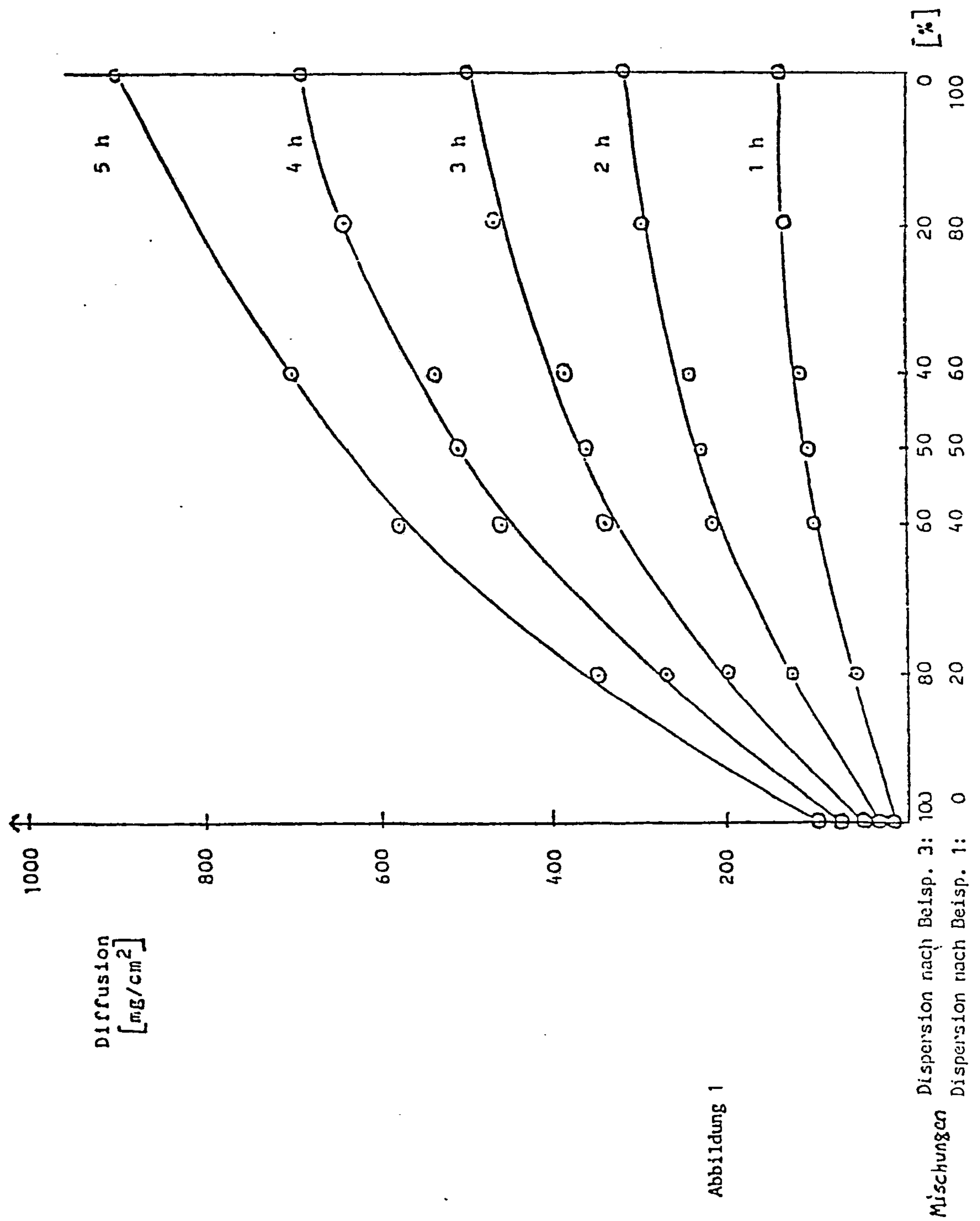
Diffusion
[mg/cm2]
1000
800
600
400
200
5 h
4 h
3 h
2 h
1 h
[%]
100 80 60 50 40 20 0
0 20 40 50 60 80 100
Mischungen
Dispersion nach Beisp. 3:
Dispersion nach Beisp. 1:

Abbildung 1